# EUROPEAN PATENT SPECIFICATION

(11) **EP 2 349 129 B1**
(45) Date of publication and mention of the grant of the patent: **04.05.2016**
(21) Application number: 09744176.0
(22) Date of filing: 12.10.2009
(51) Int. Cl.: A61F 2/91, A61F 2/915, A61F 2/90

(54) **A STENT SUITABLE FOR DEPLOYMENT IN A BLOOD VESSEL**
ZUM EINSATZ IN EIN BLUTGEFÄSS GEEIGNETER STENT
ENDOPROTHÈSE ADAPTÉE POUR LE DÉPLOIEMENT DANS UN VAISSEAU SANGUIN

(30) Priority: 10.10.2008 US 249389; 10.10.2008 EP 08253318
(43) Date of publication of application: 03.08.2011
(73) Proprietor: Veryan Medical Limited, Oxford Business Park Oxford, OX4 2HN (GB)
(72) Inventor: HERATY, Kevin, Co. Mayo (IE); MULLINS, Liam, Co. Westmeath (IE)
(74) Representative: Piésold, Alexander James
(86) International application number: PCT/GB2009/002437
(87) International publication number: WO 2010/041040

(56) References cited:
- EP-A- 0 800 801
- WO-A-03/057079
- WO-A1-00/28922
- WO-A1-2007/131798
- GB-A- 2 418 362
- US-A1- 2004 243 216

## Description

This invention relates to a stent suitable for deployment in a blood vessel to support at least part of an internal wall of the blood vessel.

WO 03/057079 relates to a braided stent with segments having different axial stiffness and/or radial stiffness. Lower axial stiffness segments are used at curved regions of a body vessel. The axial stiffness can be adjusted by changing the braid angle.

EP 0800801 discloses a stent having varying structural strength along its length. At least one end section has a thicker wall and corresponding greater radial strength than other sections of the stent, and so is more resistant to radial crushing forces.

WO 00/28922 relates to a stent with a non-uniform structure for accommodating non-uniformities in a diseased vessel. It discloses a stent constructed to provide uniform radial strength in a non-uniformly shaped e.g. tapered vessel, and a stent constructed to have varying radial strength.

WO 2007/131798 aims to provide a stent design that better copes with extreme bending. In particular, it is concerned with avoiding points of inflection of the ringlets rubbing together when the stent is bent. It teaches a stent pattern with longer struts at an end of the stent.

US 2004/243216 discloses a stent with tapered flexibility to aid deployment. A number of different stent patterns are taught that achieve this including one in which the band strut lengths decrease from one end to another of the stent.

GB 2418362 discloses a stent suitable for deployment in a blood vessel to support at least part of an internal wall of the blood vessel, the stent being movable between a delivery configuration and a deployment configuration, wherein the stent comprises a plurality of annular elements and wherein in the deployment configuration the longitudinal axis of at least a section of the stent is curved in three-dimensional space.

The present invention is characterised in that the stent comprises a first region and two second regions, the radial stiffness of the first region being greater than the radial stiffness of each of the second regions, wherein one of the second regions is located at one end of the stent, and the other second region is located at the other end of the stent; wherein the longitudinal dimension of an annular element in the first region is less than the longitudinal dimension of an annular element in each of the second regions; and wherein the stent is configured such that when it is in the deployment configuration and in a blood vessel, the diameter of the stent reduces towards each end.

Thus, the stent comprises a first region and two second regions, the radial stiffness of the first region being greater than the radial stiffness of each of the second regions. This arrangement reduces the area of the internal wall of the blood vessel which has low wall shear stress, reduces the possibility of recirculation, arid reduces the risk of neointimal hyperplasia. Wall shear stress is generated on the internal wall of a blood vessel by flow adjacent to the wall. Levels of mean wall shear stress below 0.4 Pa have been shown to have a pathogenic effect on endothelial cells which cover the inner surface of the arteries. Higher levels of wall shear stress, for example greater than 1.5 Pa, have been associated with a reduction in levels of in-stent restenosis.

Preferably the radial stiffness varies gradually from the first region towards each of the second regions.

The radial stiffness of the stent is reduced by increasing the longitudinal dimension of the annular element. Ideally the longitudinal dimension of the annular element in each of the second regions is between 1% and 90% greater than the longitudinal dimension of the annular element in the first region. Most preferably the longitudinal dimension of the annular element in each of the second regions is between 1% and 75% greater than the longitudinal dimension of the annular element in the first region. The longitudinal dimension of the annular element in each of the second regions may be approximately 40% greater than the longitudinal dimension of the annular element in the first region.

The thickness of the annular element in the first region may be greater than the thickness of the annular element in each of the second regions. In this manner the radial stiffness is also reduced by reducing the thickness of the annular element. In this specification, the term 'thickness' will be understood to mean the dimension in the radial direction.

The stent may comprise one or more connecting elements to connect a first annular element to a second annular element. Preferably the connecting element extends from the first annular element to the second annular element in a non-straight configuration. Ideally the connecting element extends from the first annular element to the second annular element in a substantially curved configuration.

The stent may comprise interconnected strut elements.

The annular element may comprise a plurality of interconnected strut elements.

Preferably the length of the strut element in the first region is less than the length of the strut element in each of the second regions. In this manner the radial stiffness is reduced by increasing the length of the strut element. Ideally the length of the strut element in each of the second regions is between 1% and 90% greater than the length of the strut element in the first region. Most preferably the length of the strut element in each of the second regions is between 1% and 75% greater than the length of the strut element in the first region. The length of the strut element in each of the second regions may be approximately 40% greater than the length of the strut element in the first region.

The width of the strut element in the first region may be greater than the width of the strut element in each of the second regions. In this manner the radial stiffness is reduced by reducing the width of the strut element. Preferably the width of the strut element in the first region is between 2% and 50% greater than the width of the strut element in each of the second regions. Ideally the width of the strut element in the first region is between 10% and 30% greater than the width of the strut element in each of the second regions. The width of the strut element in the first region is approximately 20% greater than the width of the strut element in each of the second regions.

The thickness of the strut element in the first region may be greater than the thickness of the strut element in each of the second regions. In this manner the radial stiffness is reduced by reducing the thickness of the strut element.

In one embodiment a first strut element is connected to a second strut element at a connection point. Preferably the connecting element is connected to the annular element at the connection point.

The thickness of the stent wall may be greater in the first region than in each of the second regions. For example, the stent comprises annular elements, and an annular element in the first region may have a greater thickness than an annular element in each of the second regions. Where the annular elements comprise interconnected strut elements, the strut elements in the first region may have a greater thickness than strut elements in each of the second regions.

Where an annular element is formed by interconnected strut elements, the interconnected strut elements may have a zig-zag or sinusoidal configuration. Thus a first strut element may extend longitudinally from left to right as it also extends in the circumferential direction, whilst an adjacent second strut element may extend from right to left as it also extends in the same circumferential direction. There may be a defined connection point between adjacent strut elements, e.g. in a zig-zag configuration, or a smoother transition between the two, e.g. in a sinusoidal configuration.

Preferably the stent is non-woven.

When the stent is in use, in the deployment configuration and in a blood vessel, the diameter of the stent reduces towards each end of the stent. By constructing the stent so that the diameter reduces towards an end of the stent in use, good apposition of the stent to the vessel wall may be maintained. This may help to regulate wall shear stress at the end of the stent which would otherwise occur due to a sudden change in the cross sectional area. In conventional stents, there may be a step change in the cross sectional area, i.e. a sudden increase, from an unstented part of the vessel to the stented part. This may result in recirculation of the blood flow and regions of low wall shear stress. If however the diameter of the stent reduces towards an end of the stent when it is deployed in a vessel, then these disadvantages can be mitigated, i.e. the tendency for recirculation can be reduced and regions of low wall shear stress can be minimised.

The diameter of the stent, when in the deployment configuration and in a blood vessel, preferably tapers towards the end of the stent. By providing the end region of the stent with a taper, the transition between the diameter of the unstented part of the vessel and a wider diameter in the stented part of the vessel can be made gradual.

The reducing stent diameter towards an end thereof (e.g. a gradual tapering diameter) occurs at both ends of the stent, so as to manage the transition between the unstented part of the vessel and the stented part at an inlet end and an outlet end of the stent. A reducing stent diameter at the inlet helps to manage the flow as it enters the stented part of the vessel, and is expected to be beneficial in reducing levels of in-stent restenosis. A reducing stent diameter at the outlet helps to manage the flow as it leaves the stented part of the vessel and in particular may be beneficial in reducing recirculation of the flow in the stented part, as well as improving the transition of the flow into the vessel downstream of the stent.

Because the stent diameter reduction is provided at both ends of the stent it may be possible to deploy the stent in a vessel without having to consider which end will be the inlet and which end the outlet.

The stent may have a section adjacent to the reducing diameter end section, for example a central section, which has a substantially constant diameter over the length of the section.

The reduction in stent diameter towards an end thereof may be achieved in various ways. One way is to vary the radial stiffness of the stent as discussed above. By reducing the radial stiffness of the stent towards an end thereof, when the stent is in the deployment configuration and is in a blood vessel, the reduced radial stiffness can cause the diameter of the stent to reduce towards an end thereof.

The reduced radial stiffness is achieved in a stent made up of annular elements by providing that the longitudinal dimension of an annular element at each end of the stent is less than the longitudinal dimension of an annular element further from each end.

The reduced radial stiffness of the stent may additionally be achieved by varying the stent wall thickness. The stent wall thickness may for example reduce towards an end of the stent. If the stent comprises strut elements, the reduced radial stiffness may be achieved by providing strut elements of reduced width.

With regard to the stent diameter reducing towards an end of the stent when the stent is in the deployed configuration and is in a blood vessel, the reduction in diameter may be provided by constructing the stent so that when it is in the deployment configuration and not constrained in a blood vessel, the stent diameter reduces towards the end of the stent. Thus the actual stent diameter, when not installed in a vessel, may reduce towards the end of the stent. The variation in diameter may be achieved by forming the stent with a tooling which has the same distribution of diameter as is desired on the finished stent. As the stent has curvature with the longitudinal axis of at least at least a section curved in three-dimensional space, the tooling may have the desired curvature as well as distribution of diameter.

A combination of variation in radial stiffness and variation in stent diameter could also be used to achieve the desired effect that the diameter of the stent reduces towards an end of the stent when it is in the deployment configuration and in a vessel. For example, a stent with a reduced diameter portion as well as reduced radial stiffness could be used. The reduced radial stiffness would be due to a longer longitudinal dimension of an end annular element, or additionally due to a reduced stent wall thickness (e.g. decreased strut thickness). This combination would achieve a greater tapering effect than by just varying the radial stiffness, or by just varying the diameter of the stent when not constrained in a vessel.

It is generally desirable that the tapering effect at an end of the stent should be gradual. Thus, blood may flow along an unstented part of the vessel of a given diameter, and then as it flows into the stented part at an inlet end of the stent the diameter will preferably increase gradually. At the outlet end, a taper will ensure that the vessel diameter decreases gradually towards the unstented vessel at the outlet end. The stent comprises a plurality of annular elements, and it is preferred that at least three of such elements in a longitudinal row have different properties to provide a taper. If there are only two annular elements with different properties then the change in diameter may not be as gradual as is preferred. The different properties of the annular element may be different radial stiffnesses or a combination of different radial stiffnesses and different diameters (when the stent is not constrained in a vessel).

In the deployment configuration the longitudinal axis of at least a section of the stent is curved in three-dimensional space. When the stent is deployed in the blood vessel, the stent exerts force on the blood vessel causing at least part of the longitudinal axis of the blood vessel to curve in three-dimensional space. Blood flowing through the three-dimensional curved part of the blood vessel then undergoes a swirling action. The swirling flow of blood has been found to minimise thrombosis and platelet adhesion, and to minimise or prevent coverage of the stent by ingrowth of intima. The flow pattern in the blood vessel including the swirling pattern induced by the non-planar geometry of the blood vessel operates to inhibit the development of vascular diseases such as thrombosis/atherosclerosis and intimal hyperplasia. In the deployment configuration the three-dimensional curved section may be substantially helically shaped. In the deployment configuration the three-dimensional curved section may be substantially spiral shaped. In the delivery configuration the longitudinal axis of the three-dimensional curved section may be substantially straight. Ideally in the delivery configuration the three-dimensional curved section is substantially cylindrically shaped The cylindrical shape provides a low-profile for ease of delivery.

In the deployment configuration the longitudinal axis of at least a section of the stent may be substantially straight. Most preferably in the deployment configuration the straight section is substantially cylindrically shaped

At least a section of the stent may have a helical angle which varies along the length of the section. This arrangement reduces the area of the internal wall of the blood vessel which has low wall shear stress, reduces the possibility of recirculation, and reduces the risk of neointimal hyperplasia. Preferably the helical angle varies gradually along the length of the varying helical angle section. The helical angle at one end of the varying helical angle section may be in the range of from 5° to 60°. Preferably the helical angle at one end of the varying helical angle section is in the range of from 15° to 45°. Ideally the helical angle at one end of the varying helical angle section is approximately 30°. The helical angle at the other end of the varying helical angle section may be approximately 0°. The helical angles discussed herein are those when the stent is in its deployment configuration but not constrained by being in a vessel. When the stent is in a vessel there may be a tendency for it to be straightened out and hence a reduction in the helix angle.

The stent may comprise a first end section and a second end section. Preferably the longitudinal axis of the first end section is substantially parallel to the longitudinal axis of the second end section. Ideally the longitudinal axis of the first end section is substantially co-linear with the longitudinal axis of the second end section.

The invention thus provides a stent having varying radial stiffness. The radial stiffness is less at both ends. The radial stiffness may be tapered towards an end of the stent. In this manner arterial injury at the stent ends may be reduced or avoided. The invention may also help to regulate wall shear stress at the inlet and outset which may occur due to a sudden change in cross sectional area.

The invention will be more clearly understood from the following description of some embodiments thereof, given by way of example only, with reference to the accompanying drawings, in which:
Fig. 1 is a side view of a stent according to an embodiment of the invention in a deployment configuration,
Fig. 2 is a schematic isometric view to illustrate definition of a helical angle,
Fig. 3 is a side view of part of the stent of Fig. 1 in the deployment configuration before deployment in a blood vessel,
Fig. 4 is a side view of the part of the stent of Fig. 3 in the deployment configuration after deployment in a blood vessel,
Fig. 5 is a side view of part of the stent of Fig. 1 in a delivery configuration,
Fig. 6 is a side view of part of the stent of Fig. 1 in the deployment configuration,
Fig. 7 is a side view of part of another stent deployment configuration,
Fig. 8 is a side view of part of the stent of Fig. 1 in the deployment configuration,
Figs. 9 to 13 are side views of other stents in the deployment configuration,
Fig. 14 is a side view of another stent in the delivery configuration,
Fig. 15 is a side view of the stent of Fig. 14 in the deployment configuration,
Figs. 16 and 17 are side views of other stents in the deployment configuration, and
Figs 18, 19 and 20 are side views of parts of other stents in the deployment configuration.

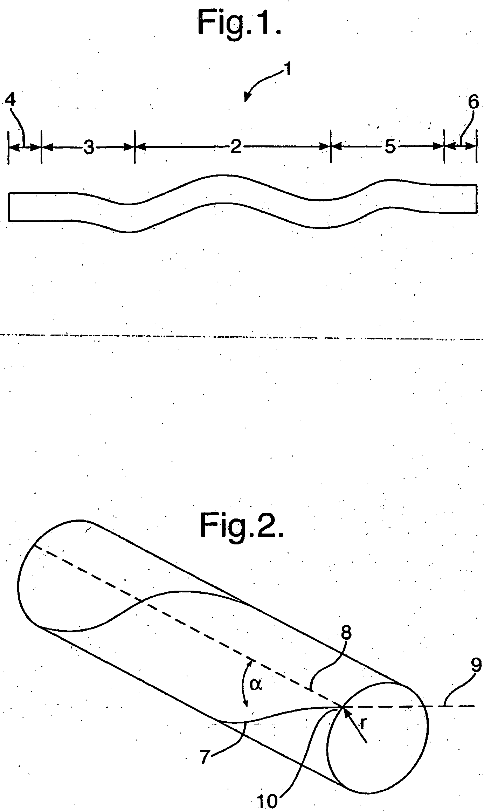

Referring to the drawings, and initially to Fig. 1 thereof, there is illustrated a stent 1 according to an embodiment of the invention. The stent 1 is suitable for deployment in a blood vessel to support at least part of an internal wall of the blood vessel.

The stent 1 comprises a central section 2, a first intermediate section 3, a first end section 4, a second intermediate section 5, and a second end section 6. The first intermediate section 3 connects the central section 2 and the first end section 4. Similarly the second intermediate section 5 connects the central section 2 and the second end section 6.

The stent 1 is movable between a collapsed delivery configuration and an expanded deployment configuration (Fig. 1). In the delivery configuration the longitudinal axis of the central section 2 is substantially straight. In particular in the delivery configuration the central section 2, the first intermediate section 3, the first end section 4, the second intermediate section 5, and the second end section 6 are all cylindrically shaped. In the deployment configuration the longitudinal axis of the central section 2 is curved in three-dimensional space. In the deployment configuration the longitudinal axis of the first end section 4 and the longitudinal axis of the second end section 6 are both substantially straight. In particular in the deployment configuration the central section 2 is helically shaped, while the first end section 4 and the second end section 6 remain cylindrically shaped (Fig. 1).

In this case the stent 1 is of a shape memory material such as Nitinol. It will be appreciated that the stent 1 may alternatively be of other materials, such as 316 L stainless steel.

In the delivery configuration and in the deployment configuration, the longitudinal axis of the first end section 4 is parallel to and co-linear with the longitudinal axis of the second end section 6, as illustrated in Fig. 1.

Fig. 1 illustrates the blended regions 3, 5, and the helical region 2. The stent and vessel centrelines are co-linear.

In an alternative arrangement, the longitudinal axis of the first end section may be parallel to and offset from the longitudinal axis of the second end section.

The first intermediate section 3 has a helical angle α which varies gradually along the length of the first intermediate section 3 from the central section 2 towards the first end section 4. Similarly the second intermediate section 5 has a helical angle α which varies gradually along the length of the second intermediate section 5 from the central section 2 towards the second end section 6. Each intermediate section 3, 5 acts as a blended region to provide a smooth transition from the helical shape of the central section 2 to the cylindrical shape of the unstented blood vessel.

The helical angle α at the central section 2 may be in the range of from 5° to 60°, preferably in the range of from 15° to 45°, and in this case is approximately 30°.

The definition of the helical angle α is illustrated in Fig. 2. Consider the helical line 7 shown in Fig. 2. Every helical line may be described by the radius r of the cylinder it lies on and the helical angle α. The helical angle α is described as the angle subtended by a parallel line 8 and a tangential line 9. The parallel line 8 is a line lying on the cylinder and parallel to the centreline of the cylinder. The tangential line 9 is a line tangential to the helical line 7 at the point of intersection 10 of the parallel line 8 and the helical line 7.

In this case the helical angle α varies from approximately 30° at the central section 2 to approximately 0° at the end sections 4, 6. The length of each intermediate section 3, 5 is approximately 22 mm in this case.

The stent diameter maybe in range of from 2 mm to 20 mm: In this case the stent diameter is approximately 6 mum.

It will be appreciated that the intermediate sections 3, 5 may have a range of helical angles and may have a range of diameters.

The definition of the helical line 7 defines the centreline path of the final forming tool geometry and therefore has a significant effect on the stent shape. The stent forming tool has a helical section towards its centre and blended regions at the proximal and distal ends. The centreline of the forming tool has a helical and blended region.

In this case the forming tool has a constant diameter and a constant cross-section over its length.

After deployment of the stent 1, the stent 1 adjusts the geometry of the blood vessel into a helical pattern. The curvature of the intermediate sections 3, 5 is matched to that of the vessel centreline, as shown in Fig. 1.

Fig. 1 illustrates the stent 1 co-linear with the blood vessel. The blended regions 3, 5 match the rate of change of centreline curvature from the helical region 2 to the straight blood vessel.

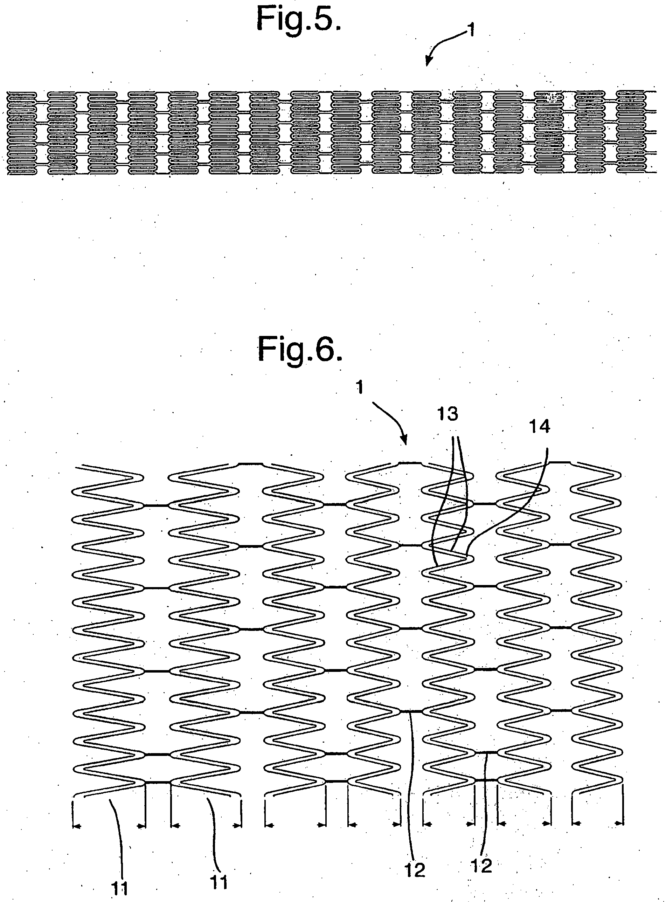

Fig. 5 illustrates the stent 1 in the collapsed state.

As illustrated in Fig. 6, the stent 1 comprises a plurality of annular elements or crowns 11, and a plurality of connecting elements 12 to connect adjacent annular elements 11.

Each annular element 11 extends around the circumference of the stent 1. Each annular element 11 comprises a plurality of interconnected strut elements 13. Adjacent strut elements 13 are connected together at connection points 14.

Each connecting element 12 may extend from a first annular element 11 to a second annular element 11 in a straight configuration, or in a curved 'Z' shaped configuration. Each connecting element 12 is connected to the annular element 11 at a connection point 14.

It will be appreciated that the stent of the invention may have a variety of possible patterns. For example the connecting element 12 may extend from a first annular element 11 to a second annular element 11 in a curved 'S' shaped configuration. The connecting element 12 between the penultimate annular element 11 and the final annular element 11 may comprise a `V' shaped portion.

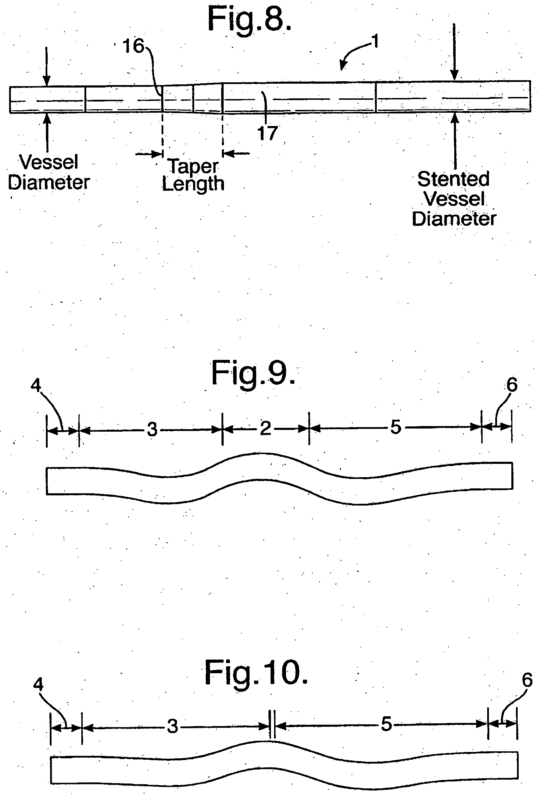

The radial stiffness of the stent 1 varies gradually along part of the length of the stent 1, as illustrated in Fig. 8. In particular the radial stiffness of the end region 16 of the stent 1 is less than the radial stiffness of a first region 17 located further in from the end of the stent 1. The radial stiffness of the stent 1 varies gradually from the first region 17 towards the end region 16.

In this case the first region 17 is located approximately 8 mm from the end region 16.

The variation in radial stiffness may be achieved by a variety of different means.

For example the longitudinal dimension of the annular element 11 in the first region 17 is less than the longitudinal dimension of the annular element 11 in the end region 16, as illustrated in Fig. 6. The longitudinal dimension of the annular element 11 in the end region 16 may be between 1% and 90% greater than the longitudinal dimension of the annular element 11 in the first region 17, preferably between 1% and 75% greater, and in this case is approximately 40% greater. Similarly the length of the strut elements 13 in the first region 17 may be less than the length of the strut elements 13 in the end region 16. The length of the strut elements 13 in the end region 16 may be between 1 % and 90% greater than the length of the strut elements 13 in the first region 17, preferably between 1% arid 75% greater, and in this case is approximately 40% greater.

Fig. 6 illustrates the variation in radial stiffness using strut length.

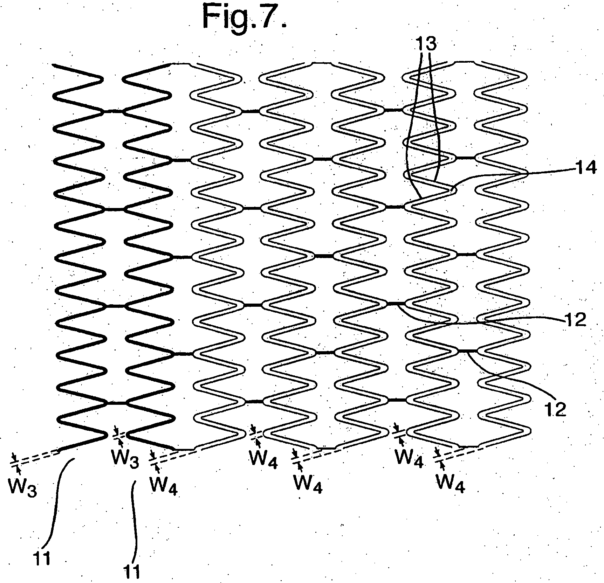

As another example the width of the strut elements 13 in the first region 17 may be greater than the width of the strut elements 13 in the end region 16, as illustrated in Fig. 7. The width of the strut elements 13 in the first region 17 maybe between 2% and 50% greater than the width of the strut elements 13 in the end region 16, preferably between 10% and 30% greater, and in this case is approximately 20% greater.

Fig. 7 illustrates the variation in radial stiffness using strut width.

As another example the thickness of the annular element 11 in the first region 17 may be greater than the thickness of the annular element 11 in the end region 16. Similarly the thickness of the strut elements 13 in the first region 17 may be greater than the thickness of the strut elements 13 in the end region 16.

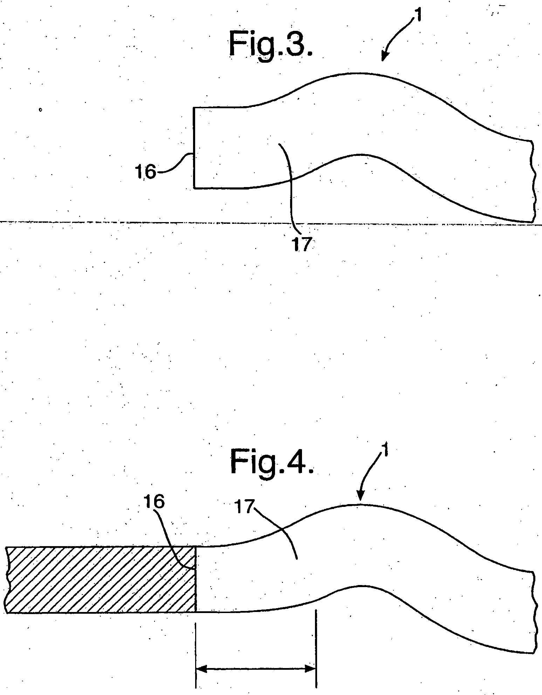

Prior to delivery when the stent 1 is outside of the blood vessel, the stent 1 has a constant diameter from the first region 17 to the end region 16, as illustrated in Fig. 3. After deployment of the stent 1 in the blood vessel, the stent 1 has a tapered configuration with a gradually reducing diameter from the first region 17 to the end region 16, as illustrated in Fig. 4, due to the variation in radial stiffness.

Fig. 3 illustrates the stent 1 before deployment with no taper evident at the stent end 16. Fig. 4 illustrates the stent 1 after deployment with the taper evident at the stent end 16.

The variation in radial stiffness along part of the length of the stent 1 reduces the area of blood vessel wall which has low wall shear stress, reduces the possibility of recirculation, and reduces the risk of neointimal hyperplasia. Levels of mean wall shear stress below 0.4 Pa have been shown to have a pathogenic effect on endothelial cells which cover the inner surface of the arteries. Higher levels of wall shear stress, for example greater than 1.5 Pa, have been associated with a reduction in levels of in-stent restenosis.

An alternative arrangement of sudden expansion from a stent to a blood vessel may lead to poor performance in terms of wall shear. The invention addresses this problem by gradually changing the diameter of the stent 1 at the ends. By varying the radial stiffness of the stent 1 the invention ensures that good apposition of the stent 1 to the vessel wall is maintained. A number of approaches are possible to achieve the diameter increase at the stent ends in a gradual manner. The strut cross section may be reduced towards the stent ends, and/or the strut length may be increased towards the stent ends.

The radial stiffness is proportional to the strut width. As the strut width increases the radial force increases. The radial stiffness is inversely proportional to the strut length. As the strut length decreases the radial force increases.

No recirculation region occurs with the stent 1 with tapering radial stiffness. A recirculation region would arise at the proximal end of a non-tapered stent with constant radial stiffness due to the sudden area change at the proximal end.

Increasing the length of the tapered region reduces the surface area of wall shear stress below 0.4 Pa.

The stent 1 has the blended regions 3, 5 and is tapered. In this manner the levels of wall shear stress are significantly improved. The regions of low wall shear stress are associated with in-stent resfonosis. Therefore increasing the wall shear stress reduces the levels of in-stent restonosis. The tapering is achieved by varying the radial stiffness at the proximal and distal ends of the stent 1.

An example includes a taper on the intermediate sections 3, 5. The taper is achieved by changing the radial stiffness. The rate of expansion of the taper is constant over the overall taper length. In this case the expansion is from a 5 mm vessel to a 6 mm stented diameter.

The effect of the taper on the blended region wall shear stress is as follows.

By including the tapering section at the proximal end of the stent 1, the region of low wall shear stress below 0.4 Pa is reduced when compared with a sudden expansion into a helical stent which would result in a large region of low wall shear stress at the proximal end of the stent due to the sudden area change from the 5 mm to 6 mm blood vessel.

The surface area below 0.4 Pa may be used as a metric to evaluate the performance of a tapered stent. Increasing the length of the taper on the blended regions 3, 5 reduces the surface area of wall shear stress below 0.4 Pa.

The first four crowns 11 may have reducing strut width. The last crown 11 may have longer strut length, thus helping to achieve the desired low radial stiffness without reducing the strut width.

In use, the stent 1 is arranged in the collapsed delivery configuration with the central section 2, the first intermediate section 3, the first end section 4, the second intermediate section 5, and the second end section 6 all cylindrically shaped. When the stent 1 is outside of the blood vessel, the stent 1 has a constant diameter from the first region 17 to the end region 16, as illustrated in Fig. 3. The stent 1 is delivered through a blood vessel to the desired site of treatment. The stent 1 is then moved from the delivery configuration to the expanded deployment configuration to support at least part of an internal wall of the blood vessel. In the deployment configuration the central section 2 is helically shaped, and the first end section 4 and the second end section 6 are cylindrically shaped. After deployment of the stent 1 in the blood vessel, the stent 1 has a tapered configuration with a gradually reducing diameter from the first region 17 to the end region 16, as illustrated in Fig. 4, due to the variation in radial stiffness.

When the stent 1 is deployed in the blood vessel, the stent 1 exerts force on the blood vessel causing at least part of the longitudinal axis of the blood vessel to curve in three-dimensional space. In this manner the stent 1 acts to support at least part of the internal wall of the blood vessel curved in three-dimensional space. Blood flowing through the three-diniensional curved part of the blood vessel then undergoes a swirling action. The swirling flow of blood has been found to minimize thrombosis and platelet adhesion, and to minimise or prevent coverage of the stent 1 by ingrowth of intima. The flow pattern in the blood vessel including the swirling pattern induced by the non-planar geometry of the blood vessel operates to inhibit the development of vascular diseases such as thrombosis/atherosclerosis and intimal hyperplasia.

It will be appreciated that the shape of the stent may be varied.

For example, as illustrated in Fig: 9, the central section 2 may be shorter in length than each of the intermediate sections 3, 5.

As illustrated in Fig. 10, the stent may comprise the first intermediate section 3, the first end section 4, the second intermediate section 5, and the second end section 6. In this case the stent does not include a central section.

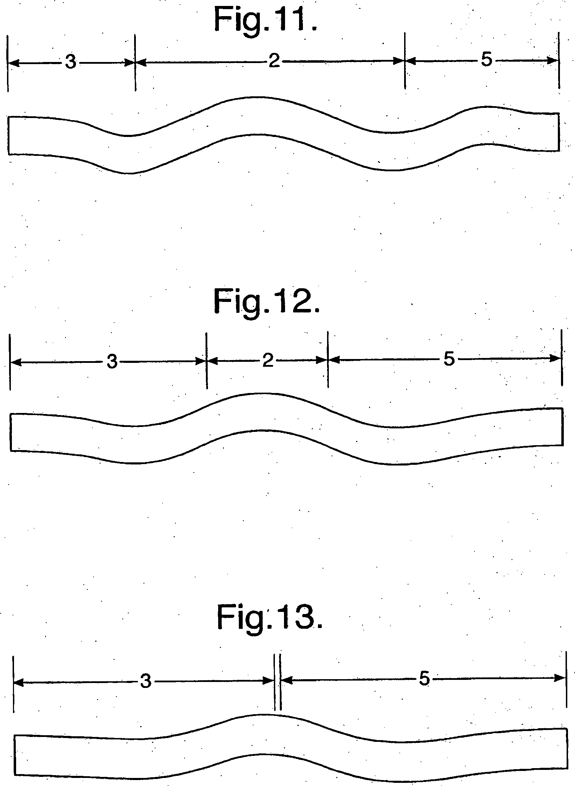

As illustrated in Fig. 11, the stent may comprise the central section 2, the first intermediate section 3, and the second intermediate section 5. In this case the stent does not include a first end section, or a second end section.

As illustrated in Fig. 12, the central section 2 may be shorter in length than each of the intermediate sections 3, 5.

As illustrated in Fig. 13, the stent may comprise the first intermediate section 3, and the second intermediate section 5. In this case the stent does not include a central section, or a first end section, or a second end section.

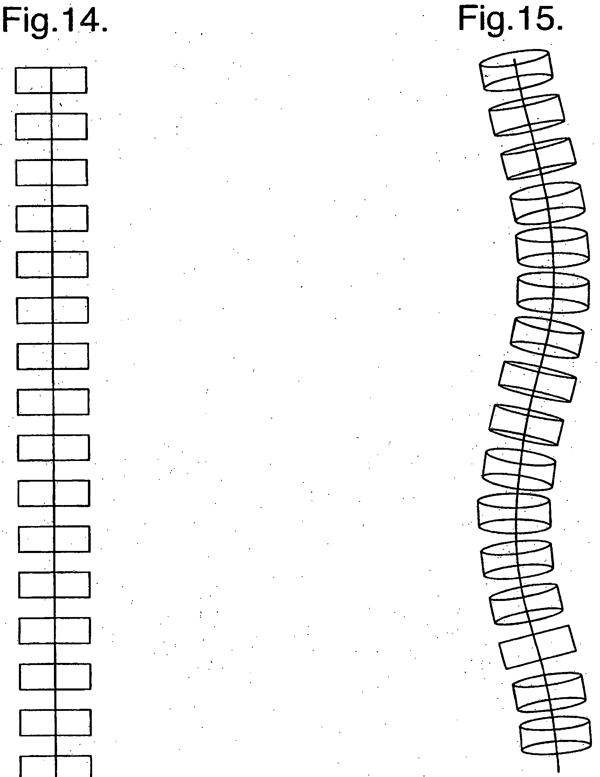

It will be appreciated that in the deployment configuration the central section may have a piecewise helical shape, as illustrated in Figs. 14 and 15. The stent may be formed of a series of annular elements in the form of short crown-shaped elements. Adjacent crowns are arranged in series and are linked by connector elements forming a tubular structure. Each crown is mostly cylindrical in shape having a straight centreline. The centreline of a straight stent is defined by a series of crown centrelines arranged in a co-linear fashion, as illustrated in Fig. 14. In certain embodiments of a three-dimensional stent, the crown centreline segments are no longer co-planar. In one such embodiment the stent centreline forms a piecewise linear three-dimensional curve. In another such embodiment the stent centreline is a series of discontinuous line segments, as illustrated in Fig. 15.

The central section may have an alternative shape, for example in the deployment configuration the central section may be substantially spiral shaped. Similarly the intermediate section may have an alternative shape, for example in the deployment configuration the intermediate section may be substantially spiral shaped.

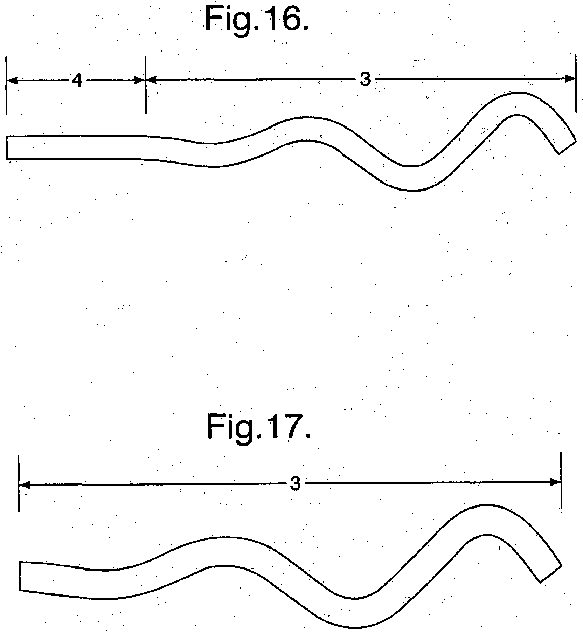

As illustrated in Fig. 16, the stent may comprise the first intermediate section 3, and the first end section 4. In this case the stent does not include a central section, or a second intermediate section, or a second end section. In the deployment configuration the first intermediate section 3 is substantially spiral shaped.

As illustrated in Fig. 17, the stent may comprise the first intermediate section 3 only. In this case the stent does not include a central section, or a first end section, or a second intermediate section, or a second end section. In the deployment configuration the first intermediate section 3. is substantially spiral shaped.

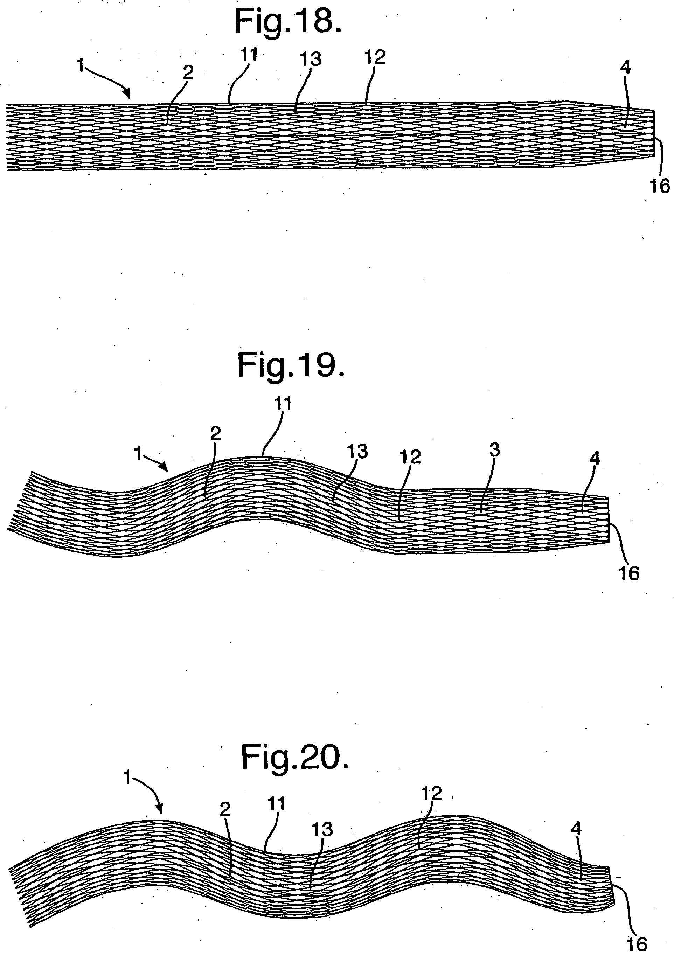

The embodiments of Figures 18, 19 and 20 are stents which when in the deployment configuration and not constrained in a blood vessel have a diameter which reduces towards the end of the stent.

The stent 1 shown in Figure 18 has a cylindrical geometry when in the deployment configuration. The stent has a central section 2 and an end section 4. The diameter of the end section 4 reduces from the diameter of the central section 2 to a smaller diameter at the end 16 of the stent. The end section 4 is thus tapered or conical. The stent 1 is made up of annular elements 11, each annular element being formed of strut elements 13 in a zig-zag shape, and adjacent annular elements being connected by connecting elements 12.

The stent 1 of Figure 19 has a central section 2, an intermediate section 3 and an end section 4. As in the case of the Figure 18 stent, the stent is made up of annular elements 11 each formed of interconnected strut elements 13, with the annular elements 11 being connected by connecting elements 12. In this case, the central section 2 has a longitudinal axis which is curved in three-dimensional space, e.g. helical. This is connected to the intermediate section 3 which is of straight cylindrical form, and section 3 is in turn connected to end section 4 which is tapered, having a reducing diameter towards the end 16 of the stent.

In the case of the stent of Figure 20, this has a central section 2 with a longitudinal axis curved in three-dimensional space, e.g. helical, which is connected to an end section 4 which is tapered. In this case, the end section 4, as well as being tapered, has a longitudinal axis which is curved in three-dimensional space.

When a stent according to Figure 18 or Figure 19 or Figure 20 is deployed in a blood vessel, its diameter will be reduced along its length by the constraining action of the vessel. At the stent end 16, however, the diameter may not be reduced at all by the constraining action, or may be reduced only slightly, thereby avoiding a sharp change in the flow lumen of the stent between the unstented part and the stented part. Rather, a gradual change is provided over the length of the end section 4.

The other ends of the stents of Figures 18 or 19 or 20 are not shown but may also have a taper and possibly the same construction as the ends which are shown.

The invention is not limited to the embodiments hereinbefore described, with reference to the accompanying drawings, which may be varied in construction and detail.

## Claims

1. A stent (1) suitable for deployment in a blood vessel to support at least part of an internal wall of the blood vessel, the stent being movable between a delivery configuration and a deployment configuration, wherein the stent comprises a plurality of annular elements (11) and wherein in the deployment configuration the longitudinal axis of at least a section of the stent is curved in three-dimensional space;
**characterised in that** the stent comprises a first region (17) and two second regions (16), the radial stiffness of the first region being greater than the radial stiffness of each of the second regions, wherein one of the second regions is located at one end of the stent, and the other second region is located at the other end of the stent; wherein the longitudinal dimension of an annular element in the first region is less than the longitudinal dimension of an annular element in each of the second regions; and wherein the stent is configured such that when it is in the deployment configuration and in a blood vessel, the diameter of the stent reduces towards each end.

2. A stent (1) as claimed in claim 1, wherein the radial stiffness varies gradually from the first region (17) towards each of the second regions (16).

3. A stent (1) as claimed in claim 1 or 2, wherein the longitudinal dimension of the annular element (11) in each of the second regions (16) is between 1% and 90% greater than the longitudinal dimension of the annular element (11) in the first region (17); preferably between 1% and 75% greater than the longitudinal dimension of the annular element in the first region; more preferably approximately 40% greater than the longitudinal dimension of the annular element in the first region.

4. A stent (1) as claimed in any preceding claim, wherein the thickness of a stent wall in the first region (17) is greater than the thickness of a stent wall in each of the second regions (16).

5. A stent (1) as claimed in any preceding claim, wherein the stent comprises one or more connecting elements (12) to connect a first annular element (11) to a second annular element (11); wherein preferably the connecting element extends from the first annular element to the second annular element in a non-straight configuration; more preferably in a substantially curved configuration.

6. A stent (1) as claimed in any preceding claim, wherein the annular element (11) comprises a plurality of interconnected strut elements (13); wherein preferably the length of the strut element in the first region (17) is less than the length of the strut element in each of the second regions (16); wherein preferably the length of the strut element in each of the second regions is between 1% and 90% greater than the length of the strut element in the first region; more preferably between 1% and 75% greater than the length of the strut element in the first region; more preferably approximately 40% greater than the length of the strut element in the first region.

7. A stent (1) as claimed in claim 6, wherein the width of the strut element (13) in the first region (17) is greater than the width of the strut element in each of the second regions (16); wherein preferably the width of the strut element in the first region is between 2% and 50% greater than the width of the strut element in each of the second regions; more preferably 10% and 30% greater than the width of the strut element in each of the second regions; more preferably approximately 20% greater than the width of the strut element in each of the second regions.

8. A stent (1) as claimed in claim 6 or 7, wherein the thickness of the strut element (13) in the first region (17) is greater than the thickness of the strut element (13) in each of the second regions (16); and/or wherein a first strut element is connected to a second strut element at a connection point (14), the connecting element (12) preferably being connected to the annular elements (11) at the connection point.

9. A stent (1) as claimed in any preceding claim, wherein, in use, when the stent is in the deployment configuration and is in a blood vessel, the diameter of the stent tapers towards each end of the stent.

10. A stent (1) as claimed in any preceding claim, wherein the diameter of the stent, when in the deployment configuration and not constrained in a blood vessel, reduces towards an end of the stent.

11. A stent (1) as claimed in any preceding claim, wherein in the deployment configuration the three-dimensionally curved section of the stent is substantially helically shaped or substantially spiral shaped.

12. A stent (1) as claimed in claim 11, wherein in the delivery configuration the longitudinal axis of the three-dimensionally curved section (2) is substantially straight, preferably substantially cylindrically shaped.

13. A stent (1) as claimed in any preceding claim, wherein in the deployment configuration the longitudinal axis of at least a section (4, 6) of the stent is substantially straight, preferably substantially cylindrically shaped.

14. A stent (1) as claimed in any preceding claim, wherein at least a section (3, 5) of the stent has a helical angle (α) which varies along the length of the section; wherein preferably the helical angle varies gradually along the length of the varying helical angle section; wherein preferably the helical angle at one end of the varying helical angle section is in the range of from 5° to 60°, more preferably in the range of from 15° to 45°, more preferably approximately 30°; wherein preferably the helical angle at the other end of the varying helical angle section is approximately 0°.

15. A stent (1) as claimed in any preceding claim, wherein the stent comprises a first end section (4) and a second end section (6); wherein preferably the longitudinal axis of the first end section is substantially parallel to the longitudinal axis of the second end section; wherein more preferably the longitudinal axis of the first end section is substantially co-linear with the longitudinal axis of the second end section.

## Patentansprüche

1. Stent (1), der dazu geeignet ist sich in einem Blutgefäß zu entfalten um mindestens einen Teil einer Innenwand des Blutgefäßes zu stützen, wobei der Stent zwischen einer Zuführungskonfiguration und einer Entfaltungskonfiguration beweglich ist, wobei der Stent eine Vielzahl von Ringelementen (11) umfasst und wobei in der Entfaltungskonfiguration die Längsachse mindestens eines Abschnitts des Stents im dreidimensionalen Raum gekrümmt ist;
**dadurch gekennzeichnet, dass** der Stent eine erste Region (17) und zwei zweite Regionen (16) umfasst, wobei die radiale Steifigkeit der ersten Region größer ist als die radiale Steifigkeit jeder der zweiten Regionen, wobei eine der zweiten Regionen sich an einem Ende des Stents befindet und die andere zweite Region sich am anderen Ende des Stents befindet; wobei die Längsabmessung eines Ringelements in der ersten Region kleiner ist als die Längsabmessung eines Ringelements in jeder der zweiten Regionen und wobei der Stent derart gestaltet ist, dass, wenn er in der Entfaltungskonfiguration und in einem Blutgefäß ist, der Durchmesser des Stents zu jedem Ende hin abnimmt.

2. Stent (1) nach Anspruch 1, wobei sich die radiale Steifigkeit von der ersten Region (17) zu jeder der zweiten Regionen (16) hin allmählich ändert.

3. Stent (1) nach Anspruch 1 oder 2, wobei die Längsabmessung des Ringelements (11) in jeder der zweiten Regionen (16) zwischen 1 % und 90% größer ist als die Längsabmessung des Ringelements (11) in der ersten Region (17), vorzugsweise zwischen 1% und 75% größer ist als die Längsabmessung des Ringelements in der ersten Region, besonders bevorzugt ungefähr 40% größer ist als die Längsabmessung des Ringelements in der ersten Region.

4. Stent (1) nach einem der vorhergehenden Ansprüche, wobei die Dicke einer Stentwand in der ersten Region (17) größer ist als die Dicke einer Stentwand in jeder der zweiten Regionen (16).

5. Stent (1) nach einem der vorhergehenden Ansprüche, wobei der Stent ein oder mehrere Verbindungselemente (12) zum Verbinden eines ersten Ringelements (11) mit einem zweiten Ringelement (11) umfasst; wobei das Verbindungselement sich vom ersten Ringelement zum zweiten Ringelement vorzugsweise in einer nicht geradlinigen Konfiguration, besonders bevorzugt in einer im Wesentlichen gekrümmten Konfiguration, erstreckt.

6. Stent (1) nach einem der vorhergehenden Ansprüche, wobei das Ringelement (11) eine Vielzahl von miteinander verbundenen Strebenelementen (13) umfasst; wobei vorzugsweise die Länge des Strebenelements in der ersten Region (17) kleiner ist als die Länge des Strebenelements in jeder der zweiten Regionen (16); wobei vorzugsweise die Länge des Strebenelements in jeder der zweiten Regionen zwischen 1% und 90% größer ist als die Länge des Strebenelements in der ersten Region; besonders bevorzugt zwischen 1% und 75% größer ist als die Länge des Strebenelements in der ersten Region; besonders bevorzugt ungefähr 40% größer ist als die Länge des Strebenelements in der ersten Region.

7. Stent (1) nach Anspruch 6, wobei die Breite des Strebenelements (13) in der ersten Region (17) größer ist als die Breite des Strebenelements in jeder der zweiten Regionen (16); wobei vorzugsweise die Breite des Strebenelements in der ersten Region zwischen 2% und 50% größer ist als die Breite des Strebenelements in jeder der zweiten Regionen, besonders bevorzugt 10% und 30% größer ist als die Breite des Strebenelements in jeder der zweiten Regionen, besonders bevorzugt ungefähr 20% größer ist als die Breite des Strebenelements in jeder der zweiten Regionen.

8. Stent (1) nach Anspruch 6 oder 7, wobei die Dicke des Strebenelements (13) in der ersten Region (17) größer ist als die Dicke des Strebenelements (13) in jeder der zweiten Regionen (16) und/oder wobei ein erstes Strebenelement an einer Verbindungsstelle (14) mit einem zweiten Strebenelement verbunden ist, wobei das Verbindungselement (12) vorzugsweise an der Verbindungsstelle mit dem Ringelement (11) verbunden ist.

9. Stent (1) nach einem der vorhergehenden Ansprüche, wobei im Einsatz, wenn der Stent in der Entfaltungskonfiguration ist und sich in einem Blutgefäß befindet, der Durchmesser des Stents zu jedem Ende des Stents hin kleiner wird.

10. Stent (1) nach einem der vorhergehenden Ansprüche, wobei der Durchmesser des Stents, wenn der Stent in der Entfaltungskonfiguration und nicht in ein Blutgefäß gezwängt ist, zu einem Ende des Stents hin abnimmt.

11. Stent (1) nach einem der vorhergehenden Ansprüche, wobei in der Entfaltungskonfiguration der dreidimensional gekrümmte Abschnitt des Stents im Wesentlichen wendelförmig oder im Wesentlichen spiralförmig ist.

12. Stent (1) nach Anspruch 11, wobei in der Zuführungskonfiguration die Längsachse des dreidimensional gekrümmten Abschnitts (2) im Wesentlichen gerade, vorzugsweise im Wesentlichen zylindrisch, geformt ist.

13. Stent (1) nach einem der vorhergehenden Ansprüche, wobei in der Entfaltungskonfiguration die Längsachse mindestens eines Abschnitts (4, 6) des Stents im Wesentlichen gerade, vorzugsweise im Wesentlichen zylindrisch, geformt ist.

14. Stent (1) nach einem der vorhergehenden Ansprüche, wobei mindestens ein Abschnitt (3, 5) des Stents einen Steigungswinkel (α) umfasst, der sich über der Länge des Abschnitts ändert; wobei sich der Steigungswinkel vorzugsweise allmählich über der Länge des Abschnitts mit sich änderndem Steigungswinkel ändert; wobei vorzugsweise der Steigungswinkel an einem Ende des Abschnitts mit sich änderndem Steigungswinkel im Bereich von 5° bis 60°, besonders bevorzugt im Bereich von 15° bis 45°, besonders bevorzugt ungefähr 30° ist; wobei vorzugsweise der Steigungswinkel am anderen Ende des Abschnitts mit sich änderndem Steigungswinkel ungefähr 0° ist.

15. Stent (1) nach einem der vorhergehenden Ansprüche, wobei der Stent einen ersten Endabschnitt (4) und einen zweiten Endabschnitt (6) umfasst; wobei vorzugsweise die Längsachse des ersten Endabschnitts im Wesentlichen parallel zur Längsachse des zweiten Endabschnitts ist; wobei besonders bevorzugt die Längsachse des ersten Endabschnitts im Wesentlichen kollinear mit der Längsachse des zweiten Endabschnitts ist.

## Revendications

1. Endoprothèse (1) appropriée pour déploiement dans un vaisseau sanguin pour supporter au moins une partie d'une paroi interne du vaisseau sanguin, l'endoprothèse étant mobile entre une configuration de pose et une configuration de déploiement, dans laquelle l'endoprothèse comprend une pluralité d'éléments annulaires (11) et dans laquelle, dans la configuration de déploiement, l'axe longitudinal d'au moins une section de l'endoprothèse est incurvé dans un espace tridimensionnel ;
**caractérisée en ce que** l'endoprothèse comprend une première région (17) et deux secondes régions (16), la rigidité radiale de la première région étant plus grande que la rigidité radiale de chacune des secondes régions, dans lesquelles une des secondes régions est située au niveau d'une extrémité de l'endoprothèse et l'autre seconde région est située au niveau de l'autre extrémité de l'endoprothèse ; dans laquelle la dimension longitudinale d'un élément annulaire dans la première région est inférieure à la dimension longitudinale d'un élément annulaire dans chacune des secondes régions ; et dans laquelle l'endoprothèse est configurée de sorte que lorsqu'elle est dans la configuration de déploiement et dans un vaisseau sanguin, le diamètre de l'endoprothèse se réduit à chaque extrémité.

2. Endoprothèse (1) selon la revendication 1, dans laquelle la rigidité radiale varie progressivement de la première région (17) vers chacune des secondes régions (16).

3. Endoprothèse (1) selon la revendication 1 ou 2, dans laquelle la dimension longitudinale de l'élément annulaire (11) dans chacune des secondes régions (16) est entre 1 % et 90 % plus grande que la dimension longitudinale de l'élément annulaire (11) dans la première région (17) ; de préférence entre 1 % et 75 % plus grande que la dimension longitudinale de l'élément annulaire dans la première région ; plus de préférence approximativement 40 % plus grande que la dimension longitudinale de l'élément annulaire dans la première région.

4. Endoprothèse (1) selon n'importe quelle revendication précédente, dans laquelle l'épaisseur d'une paroi d'endoprothèse dans la première région (17) est plus grande que l'épaisseur d'une paroi d'endoprothèse dans chacune des secondes régions (16).

5. Endoprothèse (1) selon n'importe quelle revendication précédente, dans laquelle l'endoprothèse comprend un ou plusieurs éléments de connexion (12) pour relier un premier élément annulaire (11) à un second élément annulaire (11) ; dans laquelle de préférence l'élément de connexion s'étend du premier élément annulaire au second élément annulaire dans une configuration non linéaire ; plus de préférence dans une configuration sensiblement incurvée.

6. Endoprothèse (1) selon n'importe quelle revendication précédente, dans laquelle l'élément annulaire (11) comprend une pluralité d'éléments d'entretoise interconnectés (13) ; dans laquelle de préférence la longueur de l'élément d'entretoise dans la première région (17) est inférieure à la longueur de l'élément d'entretoise dans chacune des secondes régions (16) ; dans laquelle de préférence la longueur de l'élément d'entretoise dans chacune des secondes régions est entre 1 % et 90 % plus grande que la longueur de l'élément d'entretoise dans la première région ; plus de préférence entre 1 % et 75 % plus grande que la longueur de l'élément d'entretoise dans la première région ; plus de préférence approximativement 40 % plus grande que la longueur de l'élément d'entretoise dans la première région.

7. Endoprothèse (1) selon la revendication 6, dans laquelle la largeur de l'élément d'entretoise (13) dans la première région (17) est plus grande que la largeur de l'élément d'entretoise dans chacune des secondes régions (16) ; dans lequel de préférence la largeur de l'élément d'entretoise dans la première région est entre 2 % et 50 % plus grande que la largeur de l'élément d'entretoise dans chacune des secondes régions ; plus de préférence 10 % et 30 % plus grande que la largeur de l'élément d'entretoise dans chacune des secondes régions ; plus de préférence approximativement 20 % plus grande que la largeur de l'élément d'entretoise dans chacune des secondes régions.

8. Endoprothèse (1) selon la revendication 6 ou 7, dans laquelle l'épaisseur de l'élément d'entretoise (13) dans la première région (17) est plus grande que l'épaisseur de l'élément d'entretoise (13) dans chacune des secondes régions (16) ; et/ou dans laquelle un premier élément d'entretoise est relié à un second élément d'entretoise au niveau d'un point de connexion (14), l'élément de connexion (12) étant de préférence relié à l'élément annulaire (11) au niveau du point de connexion.

9. Endoprothèse (1) selon n'importe quelle revendication précédente, dans laquelle, lors de l'utilisation, lorsque l'endoprothèse est dans la configuration de déploiement et est dans un vaisseau sanguin, le diamètre de l'endoprothèse va en s'amincissant vers chaque extrémité de l'endoprothèse.

10. Endoprothèse (1) selon n'importe quelle revendication précédente, dans laquelle le diamètre de l'endoprothèse, quand elle est dans la configuration de déploiement et non contrainte dans un vaisseau sanguin, se réduit vers une extrémité de l'endoprothèse.

11. Endoprothèse (1) selon n'importe quelle revendication précédente, dans laquelle, dans la configuration de déploiement, la section incurvée de manière tridimensionnelle de l'endoprothèse est sensiblement formée en hélice ou sensiblement formée en spirale.

12. Endoprothèse (1) selon la revendication 11, dans laquelle, dans la configuration de pose, l'axe longitudinal de la section incurvée de manière tridimensionnelle (2) est sensiblement droit, de préférence de forme sensiblement cylindrique.

13. Endoprothèse (1) selon n'importe quelle revendication précédente, dans laquelle, dans la configuration de déploiement, l'axe longitudinal d'au moins une section (4, 6) de l'endoprothèse est sensiblement droit, de préférence de forme sensiblement cylindrique.

14. Endoprothèse (1) selon n'importe quelle revendication précédente, dans laquelle au moins une section (3, 5) de l'endoprothèse a un angle hélicoïdal (α) qui varie le long de la longueur de la section ; dans laquelle de préférence l'angle hélicoïdal varie progressivement le long de la longueur de la section angulaire hélicoïdale variable ; dans laquelle de préférence l'angle hélicoïdal au niveau d'une extrémité de la section angulaire hélicoïdale variable est dans la plage de 5° à 60°, plus de préférence dans la plage de 15° à 45°, plus de préférence environ 30° ; dans laquelle de préférence l'angle hélicoïdal au niveau de l'autre extrémité de la section angulaire hélicoïdale variable est d'environ 0°.

15. Endoprothèse (1) selon n'importe quelle revendication précédente, dans laquelle l'endoprothèse comprend une première section d'extrémité (4) et une seconde section d'extrémité (6) ; dans laquelle de préférence l'axe longitudinal de la première section d'extrémité est sensiblement parallèle à l'axe longitudinal de la seconde section d'extrémité ; dans laquelle plus de préférence l'axe longitudinal de la première section d'extrémité est sensiblement colinéaire avec l'axe longitudinal de la seconde section d'extrémité.
